# EUROPEAN PATENT SPECIFICATION

(11) **EP 2 554 164 B1**
(45) Date of publication and mention of the grant of the patent: **15.04.2020**
(21) Application number: 11762059.1
(22) Date of filing: 25.03.2011
(51) Int. Cl.: A61K 9/20, A61K 9/28, A61K 31/192, A61K 31/485, A61P 29/00, A61P 25/04, A61K 45/06

(54) **PHARMACEUTICAL FORMULATION BASED ON IBUPROFEN AND CODEINE HAVING HAVING IMPROVED STABILITY**
PHARMAZEUTISCHE FORMULIERUNG AUF BASIS VON IBUPROFEN UND CODEIN MIT VERBESSERTER STABILITÄT
PRÉPARATION PHARMACEUTIQUE À BASE D'IBUPROFÈNE ET DE CODÉINE À STABILITÉ AMÉLIORÉE

(30) Priority: 30.03.2010 ES 201030479
(43) Date of publication of application: 06.02.2013
(73) Proprietor: Farmasierra Manufacturing, S.L., 28700 Madrid (ES)
(72) Inventor: TRIVES LOMBARDERO, Carmen, 28700 San Sebastian de los Reyes Madrid (ES); DEL RÍO ÁLVAREZ, Luis Alberto, 28700 San Sebastian de los Reyes Madrid (ES); SALAZAR SÁNCHEZ, Nuria, 28700 San Sebastian de los Reyes (ES); JÁUDENES SALAZAR, Eduardo, 28700 San Sebastian de los Reyes Madrid (ES); OLLEROS IZARD, Tomás, 28700 San Sebastian de los Reyes Madrid (ES); FERNÁNDEZ DE GATTA GARCÍA, Mª Rosario, 28700 San Sebastian de los Reyes Madrid (ES)
(74) Representative: Manuel Illescas y Asociados, S.L.U.
(86) International application number: PCT/ES2011/070203
(87) International publication number: WO 2011/121160

(56) References cited:
- EP-B1- 0 220 805
- WO-A1-96/05834
- WO-A1-96/05834
- DE-A1-102008 048 729
- DE-A1-102008 048 729
- ES-T3- 2 035 086
- US-A1- 2009 142 392

## Description

### TECHNICAL FIELD

The present description relates to pharmaceutical formulations based on ibuprofen and codeine as active ingredients, having improved stability and safer effect, to the procedures for preparing such formulations, as well as their use in therapy. The technical field wherein the present description lies is that of the pharmaceutical industry and, in particular, the manufacturing of drugs.

### STATE OF THE ART

Codeine, whose chemical name is 7,8-didehydro-4,5-epoxy-3-methoxy-17-methylmorphinan-6-ol, is a drug well known for being an opioid analgesic normally used in the form of a pharmaceutically acceptable salt, preferably, phosphate, normally in hydrate form for the treatment or prophylaxis of pain, particularly more severe pain.

Ibuprofen, whose chemical name is 2(RS)-2-4-(2-methylpropyl)phenyl) propionic acid, is well known as a non-steroidal anti-inflammatory drug (NSAID). It furthermore has antipyretic and analgesic action, and in this way has been used, whether in the form of the acid or as a pharmaceutically acceptable salt thereof for the treatment or prophylaxis of pain and inflammation, such as in rheumatoid arthritis, headache, neuralgia, dysmenorrhoea, to reduce the adhesion of platelets and for toothache, among other conditions. Ibuprofen is an active principle that requires relatively high doses and its compression, when preparing formulations containing this active principle, is hindered by its poor fluidity characteristics and its low fusion point. The use of an NSAID in combination with other analgesic drugs is mooted in US2009/0142392 which discloses a composition comprising an NSAID, such as ibuprofen, and paracetamol.

It has been suggested that the combination of ibuprofen and codeine has a particularly beneficial effect as an analgesic, since the sum of the analgesia obtained together with the association of both active principles is greater than that obtained independently by each one of them. European patent EP0388125 discloses the use of said combination in the treatment of acute pain, such as headache and toothache and European patent EP0535841 discloses the use of said combination in the treatment of chronic pain, such as that suffered in osteoarthritis, rheumatoid arthritis, ankylosing spondylitis, bursitis, tendinitis and cancer.

One of the problems of formulations with the form of tablets or similar comprising ibuprofen occurs during the compression phase, due to the low fusion point of said active principle. The Merck index (14th edition MS&D, WhiteHouse Station, NJ, USA) specifies that the fusion point of ibuprofen is 70°C, which means that in the typical compression processes in the pharmaceutical industry, at standard manufacturing rates of 150000 tablets/hours and with a machine effort of around 5 tons per tablet during a process lasting several hours, temperatures close to or greater than 70°C occur (ibuprofen fusion temperature), which means that traces of said drug melt and stick to the surface of the punches throughout the compression process, meaning that the tablet appearance becomes deformed.

Another problem of these formulations based on ibuprofen and codeine occurs during their storage phase, mainly when they are in the form of tablets, since with the passage of time said tablets begin to discolour and disintegrate. Therefore, the technical problems to resolve in the field of manufacturing of formulations based on ibuprofen and codeine, is the production of, on the one hand, "white" tablets, i.e. those wherein the ingredients forming them have good colour stability and do not discolour or yellow with the passage of time or due to temperature, and which equally do not disintegrate during their storage phase and, therefore, avoid the adherence of the ibuprofen to the punches during the compression phase, which gives rise to deformed tablets.

The problem of adherence to the punches during the ibuprofen compression phase is tackled by Levina M and Rubinstein (Drug Dev. Ind. Pharm., 2002, 28:5, 495-514), advising limiting the humidity content to 3.5% during the compression phase, since hydrostatic resistance is detected in the production phase due to excess humidity, which means they reduce the areas of contact and the adhesion forces between the particles, hindering the compression thereof and for this reason the need to granulate is recommended. On the other hand, Roberts et al. (J. Pharm. Pharmacol., 2004, 56:3, 299-305) analyse the influence of the lubricant magnesium stearate in the adherence of a mixture of ibuprofen and lactose to the punches during the direct compression process, revealing that the use of magnesium stearate above 1% increases the phenomenon of adherence thereto.

This problem further increases in the case of tablets based on small particles of ibuprofen when a high dose (400 mg) is required per tablet, compared with the dose typically used in pharmacopoeia (200 mg). The advantage of manufacturing tablets with high doses of ibuprofen is to facilitate the dosage regimen as, for example, only one daily tablet is necessary, compared with the two or three necessary with the current doses existing on the market. Especially in patients with memory problems, facilitating the prescription regimen is an additional advantage for their better treatment.

The problem of the synthesis of "white" tablets based on ibuprofen and codeine is insufficiently treated in American patent US4839176 which discloses a wet granulation method for ibuprofen and codeine phosphate in tablets. Nevertheless, the degradation process of the active principles starts and accelerates as there is contact of the active principles with water. European patent EP0159852 uses an alternative procedure to resolve the problem, as it mixes the active principles with a binder and a diluent but avoiding close physical contact between the active principles during the wet granulation process. The solution proposed was to use an alternative process: mixing one of the active ingredients with a binder and filling agent; wet granulating the mixture thus produced in the presence of a solvent; drying the granulated mixture; sieving the granulated mixture; and homogenizing the mixture with one or more of other pharmaceutically active components. Nevertheless, the disadvantages associated with said process in several stages are evident in themselves, as they increase the complexity of the process, the manufacturing time, the number of stages and pieces of equipment necessary, etc.

Another alternative solution to the problem of the synthesis of "white" tablets is disclosed in European patents EP0220805 and EP0535841, which propose a two-phase tablet in the first case and multi-phase in the second, wherein each active principle is separately formulated in its respective phase, preferably adding microcrystalline cellulose as a lubricant which facilitates the direct compression in patent EP0220805 and microcrystalline cellulose and croscarmellose sodium in patent EP0535841, in each one of said phases corresponding to each active principle. Moreover, DE102008048729 discloses multiparticulate tablets comprising an inner phase and an outer phase. The production of these two-phase or multi-phase formulations, respectively, in addition to guaranteeing the stability of the tablets during their storage, does not harm their availability by the patient ingesting them as part of their treatment. Nevertheless, this solution complicates and increases the price of the manufacturing process of these tablets as it requires differentiated stages for forming each phase of the final tablet.

European patent EP0274845 discloses ibuprofen and codeine formulations containing stearic acid and effective levels of the excipients cross-linked polyvinylpyrrolidone (PVP), carboxymethyl cellulose, croscarmellose sodium and especially sodium starch glycolate or mixtures thereof. These formulations were stored at 50°C and they were regularly observed to determine discolouration and swelling. Only the compositions containing calcium carboxymethyl cellulose gave satisfactory results and the other solutions showed unsatisfactory storage properties (loss of colour, degradation, swelling) in a 12-week period. This patent demonstrated the problems produced by stearic acid/stearate in the formulations based on ibuprofen and codeine as active principles, which until then it had been thought necessary to achieve a satisfactory formulation. Therefore, the only way of avoiding to that date the presence of stearic acid/stearate in the formulations was to use a formulation with several phases. In contrast, European patent EP0777477 (claiming priority of WO96/05834) discloses a single-phase formulation comprising ibuprofen and codeine, which does not include stearic acid/stearate in its composition and which furthermore has the aforementioned excipients. As lubricant it uses a hydrogenated vegetable oil, obtaining satisfactory results insofar as the tablets remain "white" and stable over time and at temperature (3 months at 40°C), but after longer times and with higher temperatures the results are no longer satisfactory.

Nevertheless, none of the solutions proposed in the state of the art, aimed at optimizing the stability of the ibuprofen and codeine tablets during their storage, resolves a problem inherent to the compression process, as it the tendency of all formulations with lubricants and other excipients such as those proposed in the state of the art: hydrogenated vegetable oil, insoluble carboxymethyl cellulose or microcrystalline cellulose salts, to have problems of adhesion to the punches and even the walls of the moulds of the compression machine. This problem decreases the performance of the tablet production process as it damages the compression punches and affects other parts of the compression machine, undesirably, as well as causing, in some cases, exfoliations in the tablets produced (capping). Obviously, the resolution of the storage and capping problems stated above cannot be in detriment of other parameters that make the formulation effectively applicable in therapy. We are referring, for example, that the excipients used to improve stability during prolonged storage of the tablets, must not delay, for example, the availability of the active principles, avoiding possible negative affections of some of the excipients selected, etc.

Proposed herein is a novel single-phase formulation based on ibuprofen and codeine, as active principles, or any of their pharmaceutically acceptable salts, in the form of tablets and similar, in association with a pharmaceutically acceptable excipient, said excipient comprising at least one diluent, a disintegrant, a fluidifying agent and a lubricant, the latter facilitating the compression process of said formulations as it avoids the formulation components from sticking to the punches of the compression machines. Additionally, the presence of the lubricant sodium stearyl fumarate in the formulations means that, for example, compared with formulations with the lubricant magnesium stearate, said formulations have better compaction, during the compression, a better and faster dissolution of the active principles from the pharmaceutical form in the organism and better stability during storage of the formulations. Furthermore, the production method of said formulation is technologically simpler than those described in the state of the art, as it manages to reduce the number of stages in its production process. The formulations described herein are more stable than those disclosed in the state of the art as they avoid the presence of incompatible substances in the preparation thereof, managing to maintain the physical and pharmacological properties of the formulation disclosed herein intact over time, not using, as has been mentioned, ingredients declared newly incompatible. These formulations are furthermore, efficacious and safe thanks to the achievement of a more integral potency than other formulations based on ibuprofen and codeine described in the state of the art, without the presence of toxic concentrations of the ingredients used in the synthesis thereof. With the term more integral potency we are referring to the fact that a drug is more potent than another the less the dose administered thereof in comparison with the second to achieve the same therapeutic action.

The technical problems present in the state of the art in the formulations containing ibuprofen and codeine having the form of tablets or similar are herein resolved from their origin, as the compatibility or incompatibility of the excipients included in said formulations are considered by by stability studies. The determination of incompatibilities between the different ingredients of the formulations provides the rational base for selecting excipients that are compatible with each active principle and thus stabilise the future drug. A risk level must be assigned relative to each excipient within their functional class and, by the design of the specific excipients, establish prototype formulations, for example, binary mixtures of ingredients at various ratios with exposure to extreme conditions. Thus, the rational prediction of the stability of the future product from the chemical structure is qualitatively possible but it is quantitatively still difficult since there are too many factors affecting the stability of the pharmaceutical product during its life cycle. Developing a stable formulation requires in-depth research in the preformulation phase with thermal studies or with different light conditions, presence/absence of water and oxidation phenomena, such as those that have been carried out herein for the development of the formulations based on ibuprofen and codeine having the form of tablets or similar disclosed therein.

Therefore, provided herein is a pharmaceutical formulation, based on ibuprofen and codeine as active principles, improved, in terms of compressibility and compatibility of all the ingredients present therein, and with a safer effect, thanks to the compatibility studies performed between the active principles and the excipients used in the synthesis thereof.

### DESCRIPTION

### Brief description

The present invention relates to a pharmaceutical formulation comprising ibuprofen or one of its pharmaceutically acceptable salts and codeine or one of its pharmaceutically acceptable salts, as defined in claims 1 to 12. Also disclosed herein is a single-phase pharmaceutical formulation based on ibuprofen and codeine (or a pharmaceutically acceptable salt of any of them), as active principles, having the form of tablets or similar, in association with pharmaceutically acceptable excipients, said excipients comprising at least one diluent, a disintegrant, a fluidifying agent and a lubricant, the latter being sodium stearyl fumarate, and which facilitates the compression of all the ingredients and avoids the effect of sticking on the punches of the compression machines. The formulation disclosed in the present invention is technologically simpler to prepare, as it decreases the number of processes and stages necessary for its production, more stable whilst effective and safe, as it does not use ingredients that we declare newly incompatible, easily compressible and more favourably coatable than those described in the state of the art.

The present invention also relates to preparation procedures of the formulation of claims 1 to 12, as defined in claims 14 and 15. In addition, the present invention relates to a pharmaceutical formulation of claims 1 to 12 for use in the treatment or prophylaxis of acute and chronic pain or inflammation, as defined in claim 13.

Also disclosed herein are the methods for preparing said compositions, as well as their uses in therapy.

The pharmaceutical formulation disclosed herein is useful in the treatment of the pain associated with chronic medical conditions such as osteoarthritis, rheumatoid arthritis, ankylosing spondylitis, seronegative arthropathies, bursitis, cold shoulder, De Quervain's disease or cancer.

The formulations of tablet type and similar, based on ibuprofen and codeine, must comply with the following parameters:
- Suitable hardness of the tablet
- Stability during storage (it does not turn yellow or disintegrate, nor cement)
- Suitable degree of humidity for compressing
- Dissolution rate of each active principle in accordance with the pharmacopoeia requirements in force
- Disintegration time of the tablet in accordance with the pharmacopoeia requirements in force
- No interaction between the active principles and the other ingredients of the formulation

To avoid the problem of sticking and lack of stability, without losing sight of the parameters that the formulations with the form of tablets or similar, based on ibuprofen and codeine, must comply with, the technique of Differential Scanning Calorimetry, or DSC, is used to avoid including in the composition of said formulations those ingredients declared incompatible using said technique. The analyses herein performed with DSC declare the compatibility of ibuprofen with the lubricant sodium stearyl fumarate and the incompatibility of ibuprofen with the lubricant magnesium stearate. As regards codeine, said active principle is herein declared incompatible with the disintegrants croscarmellose and povidone, as the formulations containing them darken over time. The incorporation of sodium stearyl fumarate in the formulation of ibuprofen and codeine in tablets or similar, did not cause a delay in the disintegration of the tablets during their prolonged storage, nor does it negatively affect the other parameters of the formulation previously mentioned.

The compressibility and disintegration analyses of the ibuprofen and codeine formulationsdisclosed herein, according to the diluent and the disintegrant used, revealed that the diluents preferably used are mannitol and pregelatinized starch and the disintegrant sodium starch glycolate.

An object of the present invention, therefore, is to provide a stable preparation having the form of tablets or similar, of the combination of ibuprofen and codeine (preferably codeine phosphate hemihydrate) which resists storage conditions of the tablets so that they do not discolour, crack, expand and maintain a level of dissolution *in vitro* of the active principles in accordance with that demanded by the European Pharmacopoeia, during the shelf life established for the product. The formulation obtained by the method disclosed herein has no degradation problems of either ibuprofen or codeine, therefore obtaining its therapeutic activity.

For the purposes of the present description, the following terms are set down:
**Eutectic point:** is the maximum temperature at which the greatest crystallization of the solvent and solute can occur. It is also defined as the lowest temperature at which the mixtures of solids A and B can melt, in the present description, ibuprofen and codeine, with a fixed composition.
**Fusion point:** is the temperature at which the solid state and the liquid state of a substance coexist in thermal equilibrium at the pressure of 1 atmosphere.
**Eutectic mixture:** is the mixture of a solvent and solute wherein, at a constant pressure, the addition of solute no longer manages to decrease the fusion point. This makes the mixture reach the lowest possible freezing point (in the case of liquids, liquefaction) and both solidify at that temperature (*eutectic temperature*).
**Safer effect or safer drug:** in the present description it is defined as a safer formulation or drug with a safer effect than that which does not produce adverse effects which are associated to the active principle and/or excipients which compose the formulation. These secondary effects may be, among others, nauseas, gastric intolerance, migraines, etc.
**Drug potency:** in the present description it is defined as the quantity or dose administered of a drug or formulation and the action it produces. Thus a formulation or drug is so much more potent than another the lower the dose administered in comparison with the second to achieve the same action.
**Single-phase formulation:** that formulation comprising the incorporation of the active principles of which it is composed in single tablet.
**Two-phase formulation:** that formulation which is composed of two differentiated parts.
**Efficacy of the formulation:** it makes reference to the maximum effect that the drug is capable of achieving. A drug will be more effective if a greater maximum effect is achieved. The efficacy is also related to the release mechanism of the active principle(s), whether slowly or at its place of greatest efficacy in the target tissue, avoiding damages to the patient due to chemical interaction, solubilizing insoluble substances, improving tastes, improving appearance, etc.
**Technologically simpler:** relates to those formulations that require a fewer number of stages in their synthesis procedure, without involving technological complexity.
**Tablet:** is a solid pharmaceutical form containing one or several active principles with therapeutic activity and excipients, formulated in size and form for a suitable use. It has great qualities of storage and safety for the patient's use. Several types of tablet or similar are distinguished such as: non-coated tablets, coated tablets, with multiple layers, lozenges, capsules, pills, etc.
**Effective therapeutic concentration:** is the minimum effective concentration above which a drug starts its therapeutic effect.
**Toxic ingredients:** are those ingredients that compose a formulation or drug and which produce adverse effects when released in the organism.
**Extragranular functions:** are the functions exercised by different ingredients which are incorporated in previously manufactured granules.

### Description of the Figures.

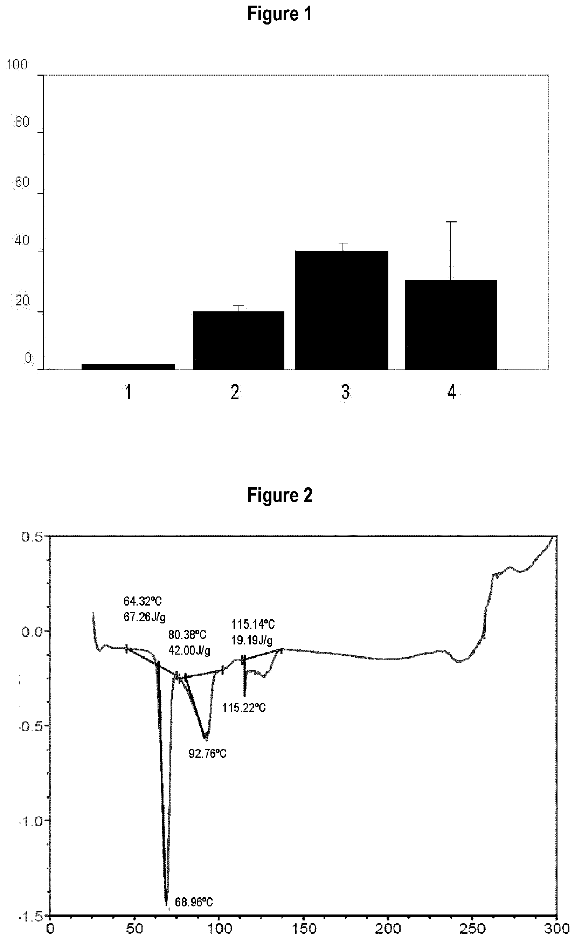

**Figure 1****. Analysis of the stability of different formulations based on ibuprofen and codeine (in the form of phosphate) together with different lubricants.** The figure shows the degree of loss of the original appearance of different formulations based on ibuprofen and codeine but which are differentiated from the lubricants that have been used in its synthesis process, as detailed:

| **Composition of the formulas** | **(1)** | **(2)** | **(3)** | **(4)** |
|---|---|---|---|---|
| Ibuprofen | 59.7% | 59.7 % | 59.7 % | 59.7 % |
| Codeine phosphate ½ H₂O | 4.5 % | 4.5 % | 4.5 % | 4.5 % |
| Mannitol | 18.1 % | 18.1 % | 18.1 % | 18.1 % |
| Pregelatinized starch | 15.7 % | 15.7 % | 15.7 % | 15.7 % |
| Sodium stearyl fumarate | 2.0 % | --- | --- | --- |
| Magnesium stearate | --- | 2.0 % | --- | 1.0 % |
| Stearic acid | | | 2.0 % | 1.0 % |
| TOTAL | 100 % | 100 % | 100 % | 100 % |

The percentage of loss of the original appearance has been studied over 1 month at a temperature of 40°C and at 75% of relative humidity. The graphic shows the degree of loss of the original appearance, in the form of percentage (Y-axis) of the formulations studied.
**Figure 2****. Thermogram of ibuprofen and sodium stearyl fumarate.** The graphic shows the thermal non-incompatibility of the mixtures with equal parts of both compounds. As observed in the graphic neither the peak corresponding to the ibuprofen (68.96°C), nor the peaks corresponding to the sodium stearyl fumarate (92.76 and 115.22°C) disappear, therefore they show no physical incompatibility for the mixture of both. The X-axis represents the temperature expressed in °C and the Y-axis represents the heat flow expressed as the thermal conductivity (W)/g.

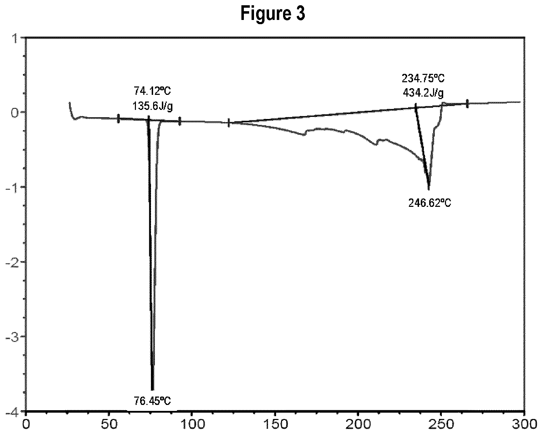

**Figure 3****. Thermogram of ibuprofen.** The figure shows the temperature peak characteristic of ibuprofen at approximately 74°C. The X-axis represents the temperature expressed in °C and the Y-axis represents the heat flow expressed as the thermal conductivity (W)/g.

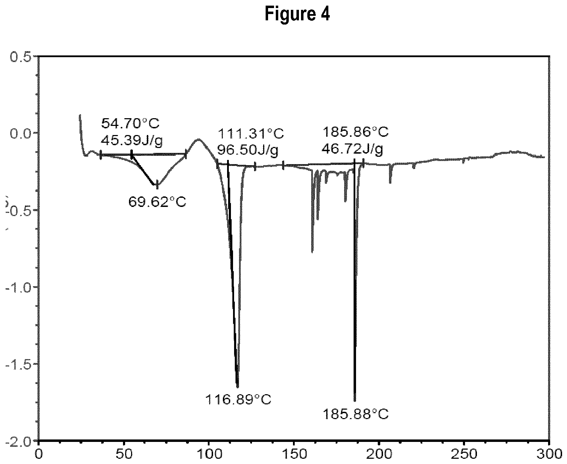

**Figure 4****. Thermogram of magnesium stearate.** The figure shows the temperature peaks characteristics of magnesium stearate at approximately 54°C, 111°C and 185 °C. The X-axis represents the temperature expressed in °C and the Y-axis represents the heat flow expressed as the thermal conductivity (W)/g.

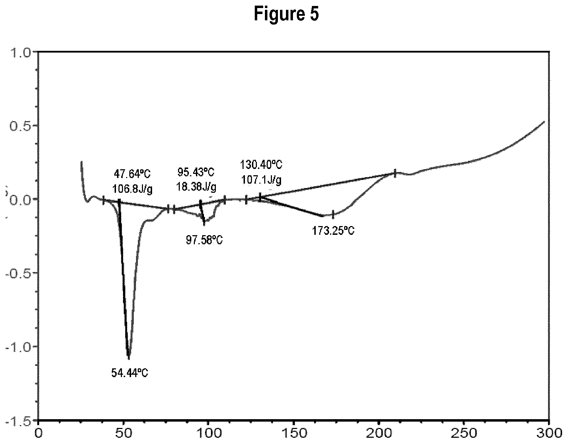

**Figure 5****. Thermogram of ibuprofen and magnesium stearate.** As can be observed in the graphic there is thermal incompatibility between the ibuprofen and magnesium stearate due to the physical disappearance of the peak characteristic of ibuprofen at 74°C. The X-axis represents the temperature expressed in °C and the Y-axis represents the heat flow expressed as the thermal conductivity (W)/g.

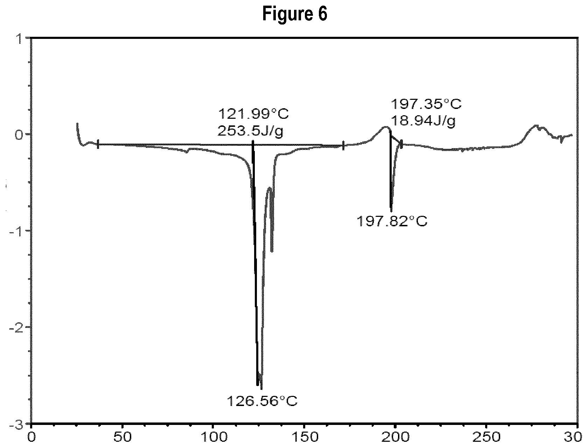

**Figure 6****. Thermogram of sodium stearyl fumarate.** The figure shows the temperature peaks characteristics of sodium stearyl fumarate at approximately 121°C and 197°C. The X-axis represents the temperature expressed in °C and the Y-axis represents the heat flow expressed as the thermal conductivity (W)/g.

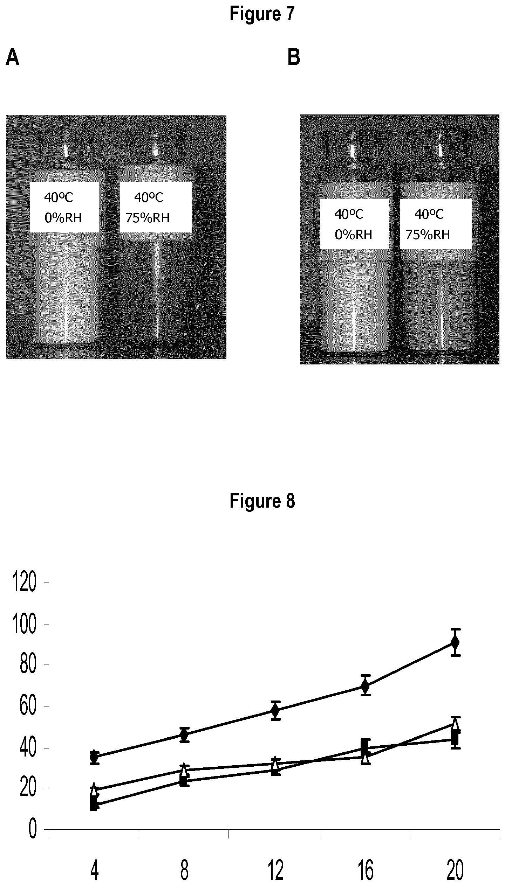

**Figure 7****. Visual analysis of incompatibility in mixtures of codeine (as phosphate hemihydrate) with the disintegrants povidone (A) and croscarmellose sodium (B) subjected during 6 months to 40°C and with 0% or 75% of relative humidity.** The photographs show a darkening of the mixtures with povidone and with croscarmellose sodium, which implies incompatibility with said two excipients.
**Figure 8****. Analysis of compressibility of formulations based on ibuprofen and codeine (phosphate) according to the type of diluent used in the preparation thereof.** The graphic shows the compressibility of three formulations based on ibuprofen and codeine with different diluents added in 50% over the final weight of the formulation: mannitol (◆), lactose (■) and microcrystalline cellulose PH101 (△). The X-axis represents the compression force expressed in KiloNewton(KN) and the Y-axis represents the breaking force expressed in Newton (N).

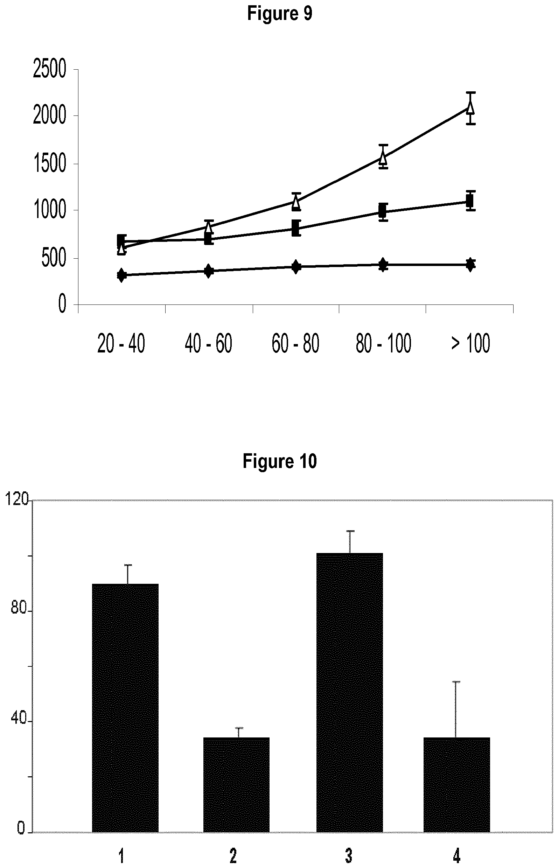

**Figure 9****. Analysis of disintegration of formulations based on ibuprofen and codeine (phosphate) according to the type of diluent used in the preparation thereof.** The graphic shows the disintegration of three formulations based on ibuprofen and codeine with different diluents added in 50% over the final weight of the formulation: mannitol (◆), lactose (■) and microcrystalline cellulose PH101 (△). The X-axis represents the breaking force (N) and the Y-axis represents the RFE (Royal Spanish Pharmacopeia) disintegration time expressed in seconds.
**Figure 10****. Analysis of compressibility of formulations based on ibuprofen and codeine (phosphate) according to the type of disintegrant used in the preparation thereof.** The graphic shows the compressibility of four formulations based on ibuprofen and codeine with different disintegrants added in 10% over the final weight of the formulation: starch glycolate (1), crospovidone (2), pregelatinized starch (3) and corn starch (4). And the Y-axis represents the breaking force expressed in Newton (N).

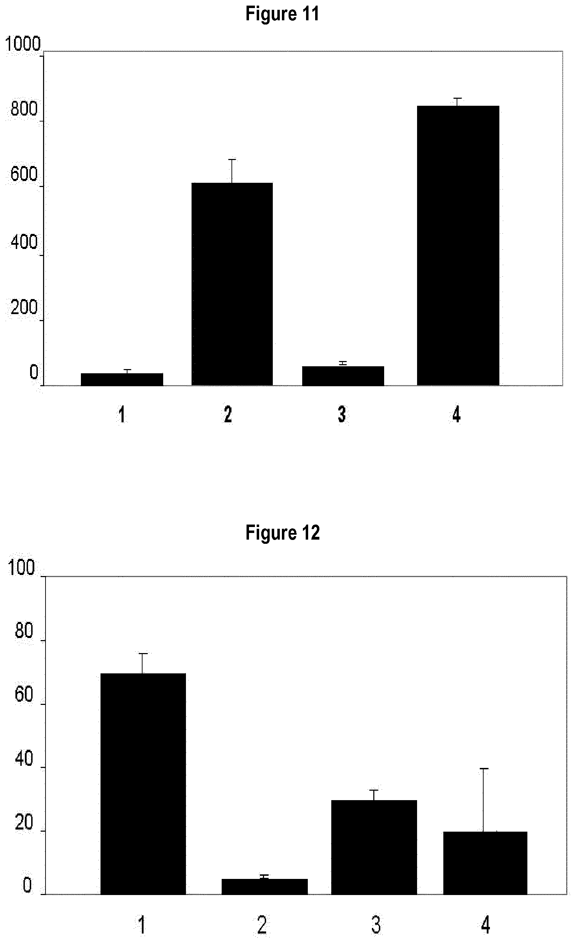

**Figure 11****. Analysis of disintegration of formulations based on ibuprofen and codeine (phosphate) according to the type of disintegrant used in the preparation thereof.** The graphic shows the disintegration of four formulations based on ibuprofen and codeine with different disintegrants added in 10% over the final weight of the formulation: starch glycolate (1), crospovidone (2), pregelatinized starch (3) and corn starch (4). And the Y-axis represents the disintegration time expressed in seconds.
**Figure 12****. Analysis of the stability of different formulations based on ibuprofen and codeine (in the form of phosphate) according to its finish or coating.** The stability of the different formulations has been analysed during a period of 6 months at a temperature of 40°C and with 75% relative humidity. Formulation 1 has no coating, formulation 2 has a blue-coloured coating (Sepifilm® LP 761 and Sepisperse® dry 1034 blue), formulation 3 has a white-coloured coating (any type of coating in the absence of Sepisperse® dry) and formulation 4 has a yellow-coloured coating (Sepifilm® LP 761 and Sepisperse® dry 3024 yellow). And the Y-axis represents the degree of change of colour expressed as percentage.

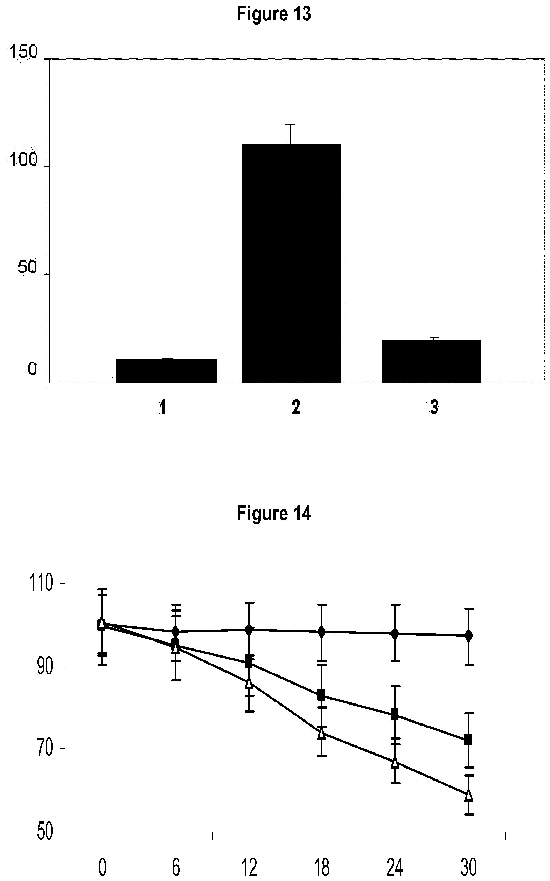

**Figure 13****. Analysis of compactibility of different formulas of ibuprofen and codeine (phosphate) according to the type of compression procedure they are subjected to.** Formulation 1 was subjected to the direct compression procedure. Formulation 2 was subjected to the dry granulation procedure and composition 3 was subjected to the wet granulation procedure. And the Y-axis represents the breaking force expressed in Newton (N).
**Figure 14****. Analysis of the potency of different formulations based on ibuprofen and codeine (phosphate) over time.** The graphic shows the analysis of the potency, expressed as the percentage of richness in ibuprofen/codeine phosphate (Y-axis), of 3 different ibuprofen and codeine formulations stored during 30 months at 25°C and with 60% relative humidity. The symbol ◆ corresponds to the formulation synthesized in accordance with example 4. The symbol ■ corresponds to the formulation of example 4 but without lubricant (sodium stearyl fumarate). The symbol △ corresponds to the formulation of example 4 but wherein the lubricant of said formulation has been change for magnesium stearate. The X-axis represents the time expressed in months. And the Y-axis represents in the form of percentage the degree of richness of the composition (ibuprofen/codeine phosphate).

### Detailed description

The present invention relates to a pharmaceutical formulation as defined in claims 1 to 12. Also disclosed herein are pharmaceutical formulations in the form of tablets or similar, comprising a combination of ibuprofen and codeine (or any of their pharmaceutically acceptable salts), in association with a pharmaceutically acceptable excipient, said excipient comprising at least a diluent, a disintegrant, a fluidifying agent and a lubricant, characterized in that the lubricant is sodium stearyl fumarate. The formulation of the invention is technologically simpler, as it decreases the number of processes and stages necessary for its production without involving greater technological complexity and with better efficacy, safety and stability, as it does not use excipients declared incompatible with the active principles of the formulation (demonstrated by DSC techniques), than the formulations based on ibuprofen and codeine described in the state of the art.

Also disclosed herein are the methods for preparing said compositions, as well as their use in therapy. Thus, the present invention also relates to preparation procedures of the formulation of claims 1 to 12, as defined in claims 14 and 15. In addition, the present invention relates to a pharmaceutical formulation of claims 1 to 12 for use in the treatment or prophylaxis of acute and chronic pain or inflammation, as defined in claim 13.

The pharmaceutically acceptable salts are those typically used in the state of the art. Thus, sodium or lysine salts are typical for ibuprofen. In the case of codeine, the salts include hydrochloride, sulphate, and, more preferably, phosphate. The preferred combination uses the acid form of ibuprofen, and codeine phosphate. It must be remembered that codeine phosphate is under the form of hemihydrate.

For the oral administration, the drug must be ingested in the form of tablets, coated tablets, capsules, lozenges, pills and granulated or non-granulated powders.

The ibuprofen and codeine can be present, for example, in the form of a previously mentioned pharmaceutically acceptable salt in any therapeutically effective quantity. For example ibuprofen or a pharmaceutically acceptable salt thereof can be present at a unitary dose of 250 to 700 mg (expressed in weight of the free acid) of ibuprofen, preferably 400 mg, and codeine or a pharmaceutically acceptable salt thereof can be present at a unitary dose of 20 to 40 mg (expressed in weight of the free anhydrous base) of codeine, preferably 30 mg in the form of codeine phosphate hemihydrate.

The formulations are administered so that they do not exceed the maximum daily dose in humans for any of the two active principles. All the quantities of ingredients or weights of the components given herein are subject to the usual pharmaceutically acceptable tolerances. Said tolerances are accepted as ± 5%. For example, the preferred quantities of ibuprofen or codeine vary from 95-105% of the figures specified.

Thus, the first object of the present description is a pharmaceutical formulation in the form of tablets or similar comprising ibuprofen or one of its pharmaceutically acceptable salts and codeine or one of its pharmaceutically acceptable salts, in combination with a pharmaceutically acceptable excipient, said excipient comprising at least one diluent, a disintegrant, a fluidifying agent and a lubricant characterized in that the lubricant is sodium stearyl fumarate.

In a preferred embodiment, the pharmaceutical formulation of the description is characterized in that the diluent is preferably added at a concentration of between 10-50% w/w over the total weight of the formulation and it is preferably selected from lactose, monocrystalline cellulose, sorbitol, mannitol pregelatinized starch and various calcium phosphates, or mixtures thereof; preferably using mannitol and pregelatinized starch. The mannitol is preferably used at a concentration of 20% w/w over the total weight of the formulation and the pregelatinized starch is preferably used at a concentration of 10% over the total weight of the formulation.

In another preferred embodiment, the pharmaceutical formulation of the description is characterized in that the disintegrant is preferably added at a concentration of between 2-20% w/w over the total weight of the formulation and it is preferably selected from crospovidone, insoluble polyvinyl povidone and sodium starch glycolate, or mixtures thereof, preferably using sodium starch glycolate.

In another preferred embodiment, the pharmaceutical formulation of the description is characterized in that the fluidifying agent is preferably added at a concentration between 0.1-1% w/w over the total weight of the formulation and it is preferably selected from talc and colloidal anhydrous silica or mixtures thereof.

In another preferred embodiment, the pharmaceutical formulation of the invention is characterized in that the sodium stearyl fumarate is preferably added at a concentration ≥ 2% over the weight of the formulation.

In another preferred embodiment, the pharmaceutical formulation of the invention is characterized in that it is free from povidone and croscarmellose sodium.

In another preferred embodiment, the pharmaceutical formulation of the description is characterized in that it consists of a core comprising ibuprofen and codeine or one of their salts in combination with at least one diluent, a disintegrant, a diluent and sodium stearyl fumarate as lubricant, coated by a film coating.

In another preferred embodiment, the pharmaceutical formulation of the invention is characterized in that the film coating is preferably formed by at least one film-forming polymer, a plasticizing agent and an opacifying or colouring agent, or mixtures thereof. The film coating involves an increase in the weight of the formulation preferably between 1-5% w/w over the total weight of the formulation, more preferably an increase of 3% w/w.

In another preferred embodiment, the pharmaceutical formulation of the description is characterized in that the film-forming polymers are preferably selected from modified cellulose ethers and derivatives of acrylic and methacrylic acids, preferably using modified cellulose ethers, such as hypromellose or hydroxypropyl methylcellulose.

In another preferred embodiment, the pharmaceutical formulation of the description is characterized in that the plasticizing agent is preferably found at a ratio with the film-forming agent of 1/4 to 1/8, that ratio preferably being 1/6 and it is preferably selected from macrogol 4000, macrogol 6000 or diethyl citrate.

In another preferred embodiment, the pharmaceutical formulation of the description is characterized in that the opacifying or colouring agent is preferably found at a ratio with the film-forming agent of 1/1 to 1/4, preferably being titanium dioxide and preferably at a ratio of 1/3 and it is selected from different commercial opacifiers.

In another preferred embodiment, the pharmaceutical formulation of the invention is characterized in that it preferably comprises a concentration of 250 mg to 750 mg of ibuprofen, more preferably 400 mg and a concentration preferably of 20 mg to 40 mg of codeine as hemihydrate phosphate, more preferably 30 mg, per dosage unit.

In another preferred embodiment, the pharmaceutical formulation of the description is characterized in that it is preferably in the form of tablets or similar, the tablets preferably being single-phase and coated single-phase tablets.

The pharmaceutical formulation of the invention is characterized in that the pharmaceutically acceptable excipients are mannitol, pregelatinized starch, sodium starch glycolate, colloidal anhydrous silica and sodium stearyl fumarate.

Another object of the present description relates to the use of the pharmaceutical formulation described above in the treatment or prophylaxis of acute and chronic pain or inflammation, preferably of headache, neuralgia, dysmenorrhoea, toothache, osteoarthritis, rheumatoid arthritis, ankylosing spondylitis, bursitis, cold shoulder, tendinitis and pain associated with cancer.

Another object of the present description relates to the method of treatment or prophylaxis of acute and chronic pain or inflammation consisting of the oral administration to a patient of an effective quantity of the pharmaceutical formulation.

Another object of the present invention relates to the preparation procedure of the formulation of claims 1 to 12 described above characterized in that it can be selected from direct compression as disclosed in claim 14 and dry granulation as disclosed in claim 15, preferably using the dry granulation method.

In an embodiment, the direct compression procedure of the present invention consists of:
Premixing phase:
   a) Pulverization and screening of the codeine.
   b) Screening of the ibuprofen together with the excipients, with the exception of the lubricant, through a sieve with appropriate mesh size.
Mixing phase:
   a) Mixing the pulverized and screened codeine in the previous step together with the other ingredients, ibuprofen and excipients, with the exception of the lubricant, in an appropriate mixer.
   b) Adding the lubricant.

Compression phase: compressing the mixture obtained in the previous step, preferably in a rotary machine.

And furthermore optionally the cores of tablets of the previous stage can be coated with a film, preferably by the application in the form of aerosol of a preferably aqueous suspension.

In another preferred embodiment, the dry granulation procedure of the present invention consists of:
Compaction phase: Mixing the ibuprofen with a disintegrant and subjecting them to a suitable pressure for their compaction obtaining briquettes.
Regularization phase: Subjecting the briquettes obtained in the previous step to a regularization procedure in a regularizing machine until obtaining granulated ibuprofen.
Premixing phase: Adding the codeine and the other excipients, with the exception of the lubricant, to the granulated ibuprofen obtained in the regularization phase and then screening it.
Mixing phase: Adding the lubricant to the mixture obtained in the premixing phase.
Compression phase: Compressing the mixture obtained in the mixing phase in a compression machine.

And furthermore optionally the cores of tablets of the previous stage can be coated by the application of a suspension, preferably in the form of aerosol of a preferably aqueous suspension.

The following examples serve to illustrate the invention, and disclose a preferred quantitative and qualitative composition of the invention and the satisfactory assays measured about the stability parameters of the tablet and dissolution profile of the active principles, ibuprofen and codeine, contained in the formulation. The examples must not be considered as limiting of the scope of the invention and other procedures will be evident for those persons skilled in the art.

### EXAMPLES

### Example 1. Detection of incompatibilities between the ingredients

To detect incompatibilities between the active principles and the excipients that will form part of the formulation described herein, various binary mixtures are made of the drugs with different excipients commonly used in the state of the art, to visually observe the colour or analyse using specific assays the modification of their thermal properties using the DSC technique when said ingredients are mixed, and thus know what excipients are incompatible with the active principles. The excipients and active principles with chemical interaction will be those in whose mixture causes at least one colour different to the original or a change occurs in the fusion or eutectic points of the two kinds of batch, in both cases due to the appearance of a novel chemical species of different properties.

### 1.- Ibuprofen

Herein it has been surprisingly revealed that, when in the compression phase sodium stearyl fumarate is added as lubricant to the mixture of ibuprofen and codeine, highly satisfactory results are obtained insofar as the tablets remain white and stable over time at the temperature tested (1 month at 40°C and 75% relative humidity), in contrast with other formulations based on ibuprofen and codeine, but containing fat lubricants (magnesium stearate, stearic acid and mixture of both), as shown in Figure 1. Therefore, and in contrast to what is suggested in the state of the art, it is possible to prepare a single-phase formulation without using stearic acid/stearate, which is directly compressible and which overcomes the aforementioned stability and storage problems associated with the presence of stearic acid/stearate in the formulations based on ibuprofen and codeine, thus including sodium stearyl fumarate as lubricant resolves the previously described compression and storage problems.

The technique called Differential Scanning Calorimetry or DSC has been used for the thermal detection of possible incompatibilities among ingredients of the pharmaceutical formulation described herein. Thus, by using a differential Scanning Calorimeter, DSC-7 (Perkin-Elmer, USA) at a heating rate of the samples of 5°C/min, the quantity of energy or enthalpy required to keep the sample at the same temperature as that of the reference and that at each of the test temperatures is measured. If no physical or chemical changes occur in the sample, the temperature does not vary nor does it consume energy to maintain an isotherm. However, when phase changes occur, the isothermal energy required is registered as an electric signal generated by thermocouples. It is understood that a change of phase occurs (or appearance of incompatibility) if the expected temperature value varies above the considered environment by 10 % with respect to its original. Said error is carried from that produced typically in the analytical measurements (± 5%, typical in terms of reproducibility), the uncertainty of apparatus calibration (± 1%), the accuracy of the micromixture of ingredients achieved (± 3%), and the rapid heating rates (± 1% for heating >1°C/min). DSC is widely used in the industry as a quality control instrument due to its applicability in assessing the purity of the samples and studying the curing of the polymers. Using said technique it has been observed that the mixture of ibuprofen with one of the lubricants most used in the state of the art as is magnesium stearate (Figure 5), makes the fusion point of ibuprofen decrease from 74°C (Figure 3) to 54.4°C, due to the formation of an incompatible eutectic mixture. This fact is easily verified from the binary thermogram of ibuprofen (Figure 3) where a characteristic peak of ibuprofen is observed at 74.12°C (135.6 J/g) which corresponds to its fusion point (75-78°C) and another not very defined (125-270°C approximately) of products of its decomposition; and of magnesium stearate (Figure 4) with a peak at 69.6°C (45.4 J/g) attributable to stearic acid, another at 116.9°C (96.50 J/g) as magnesium stearate, and another at 185.9°C (46.72 J/g) of magnesium palmitate since under the trade name of magnesium stearate a mixture is usually found principally composed of stearate and magnesium palmitate. In the simple physical mixture in equal parts of ibuprofen and magnesium stearate (Figure 5), a first peak is observed with a maximum of 54.4°C due to the formation of an eutectic of ibuprofen with stearic acid as a novel substance with greater ease of sticking in productive processes to the punches of compression machines. The other the peaks, although advanced, correspond to the fusion of magnesium stearate (97.6°C/18.4 J/g), magnesium palmitate (173.3°C/107.1 J/g) and products of the thermal decomposition of ibuprofen.

Nevertheless, it is verified from the thermogram of sodium stearyl fumarate (Figure 6), defined in its thermogram by the appearance of a peak at 121.99°C/253.5 J/g and another of its by-products at 197.35°C/18.9 J/g which in the study of a mixture in equal parts with ibuprofen (Figure 2), does not show any physical incompatibility for ibuprofen understanding that it avoids in the productive processes that the mixture sticks to the punches in compression machines. This leads to the peaks corresponding to the fusion of ibuprofen (68.96°C / 67.26 J/g) and sodium stearyl fumarate (92.76°C/42.0 J/g and 115.22°C/19.19 J/g) being only slightly advanced (but chemometrically admissible) and that it maintains the enthalpy of fusion in ibuprofen proportional to that performed with the raw material separately. The disappearance of the last peak of sodium stearyl fumarate (expected at 197.8°C) is not significant affecting the anticipated fusion of ibuprofen.

During the punch adherence tests of the compacting machines performed with the different synthesized formulations of ibuprofen and codeine (phosphate) in accordance with the present invention, the best results have been obtained using only 2% w/w of sodium stearyl fumarate in the formulation as lubricant. The results demonstrate the lower degree of adhesion to the punches of the formulations that included sodium stearyl fumarate in 2% w/w of the formulation as lubricant, whilst the formulations with magnesium stearate in a percentage of 2% w/w showed a degree of adhesion to the punches of the compacting machines of approximately 70%.

### 2.- Codeine and ibuprofen

As regards the incompatibilities of codeine (as codeine phosphate hemihydrate), the visual examination or analyses of phases thereof in internationally recognized forced conditions 6 months at 40°C and 75% relative humidity has been valuable (with its control in the absence of relative humidity) (Figure 7). In the presence of disintegrating agents such as povidone (chemically linear polymer of 1-ethylene-pyrrolidin-2-one) and croscarmellose sodium (chemically, sodium salt of a partially cross-linked cellulose in *O*-methyl bonds) (Figure 7), a darkening is observed in the mixtures of ibuprofen and codeine, which implies an incompatibility of said two excipients with the active principles ibuprofen and codeine. Both excipients are widely used in formulations of pharmaceutical products in general and formulations which have ibuprofen and codeine as active principles, in particular, but as is shown herein, said disintegrants are incompatible with the formulations based on ibuprofen and codeine, as they cause a darkening thereof.

Therefore, and in accordance with the preliminary date presented herein, a formulation is obtained having the form of tablets or similar based on ibuprofen and codeine (or a pharmaceutically acceptable salt of any of them) as active principles in association with a pharmaceutically acceptable excipient, said excipient comprising at least one lubricant, a disintegrant and a diluent characterized in that the lubricant is preferably solid stearyl fumarate and it is added preferably at a concentration ≥ 2%, and the disintegrant cannot be either povidone or croscarmellose sodium.

### 3.- Compression diluents: excipients used to favour the compression of active principles that by themselves lack the capacity to compact after the application of an extreme force.

Diluents are largely responsible for the compression phenomenon. The present invention uses a diluent which is directly compressible, i.e. it has good compression characteristics, in addition to favouring the compression of other components present in the mixture.

Compressibility tests were performed in already finished formulations of ibuprofen and codeine together with different diluents at 50% (mannitol, lactose and microcrystalline cellulose PH101 or mixtures thereof). The compressibility tests were performed in accordance with the pressure of the machine exercised on the bulk (expressed as KN) and the hardness of the tablets obtained (expressed in N). Figure 8 shows the best results obtained in terms of compressibility (or more resistant or harder tablets) were obtained in the formulations that included mannitol as diluent compared with the formulations with lactose or microcrystalline cellulose (Figure 8). These compressibility results of the formulations that include mannitol as diluent do not compromise the ease the tablets have of quickly disintegrating, these formulations even being those that use least disintegration time to exercise their expected pharmaceutical efficacy or action, therefore having a greater efficacy than the other formulations comprising other diluents (Figure 9).

Thus, diluents are preferably used herein at concentrations of between 10-50% w/w over the total of the formula and which are selected from lactose (chemically, O-b-D- galactopyranosyl-(1-4)-a-D-glucopyranose monohydrate), pregelatinized starch, microcrystalline cellulose (partially depolymerized cellulose), sorbitol, mannitol and various calcium phosphates (of the type of Emcompres® from JRS Pharma, Germany), or mixtures thereof. Mannitol is preferred of the brand Pearlitol® 200 SD from Roquette SA (France) of improved properties for tablets preferably at a dose of 20% w/w over the total weight of the formula and/or pregelatinized starch of brand Starch 1500® from Colorcon Co (USA) preferably at a concentration of 10%.

### 4.- Disintegrants

Disintegrants contribute to the fast disintegration of tablets or formulations in the gastrointestinal fluid. The formulation described herein also comprises a disintegrant which is selected from corn starch, crospovidone or insoluble polyvinyl povidone, pregelatinized starch and a starch glycolate salt such as sodium starch glycolate or mixtures thereof and which are included in a proportion of 2-20% w/w over the total weight of the formula.

As in the case of the analysis of the diluents, compressibility and disintegration tests were performed on already finished formulations of ibuprofen and codeine containing different disintegrating agents (Figure 10) at a percentage of 10% w/w such as: sodium starch glycolate (formulation 1), crospovidone (formulation 2), pregelatinized starch (formulation 3) and corn starch (formulation 4). The best results in terms of compressibility of ibuprofen and codeine formulas according to the type of disintegrant were obtained in the formulations comprising pregelatinized starch (Figure 10). Likewise, the disintegration tests with said formulations demonstrated that the tablets obtained with the mixture of ibuprofen and codeine with pregelatinized starch had very short disintegration times (Figure 11).

Thus, herein disintegrants are preferably used at concentrations of 2-20% w/w over the total of the formula and that they are selected from crospovidone or insoluble polyvinylpyrrolidone, due to their cross-linked bonds, from the brand Kollidon® CL (BASF, Germany), pregelatinized starch and a starch glycolate salt such as sodium starch glycolate (Explotab® from JRS Pharma, Germany) and mixtures thereof. More advantageously it uses pregelatinized starch (Starch 1500® from Colorcon Co, USA) preferably at a concentration of 10% w/w over the total of the formula to thus achieve a faster therapeutic effect of establishment without compromising the excellent compressibility (Figure 10). Said better availability of both active principles is demonstrated in various ibuprofen and codeine formulations (phosphate) with the most favourable disintegration (Figure 11).

### 5.- Fluidifying agents.

Other ingredients can be added to the formulation such as those conventionally used in the state of the art, for example a fluidity enhancer or fluidifying agent. As characteristic agents that improve the flow of powders and the rate of the compression process, talc is preferably used or colloidal anhydrous silica also called colloidal silicon dioxide (Aerosil® 200 from Degussa, Germany), in concentrations preferably of 0.1-10% w/w over the total of the formulation, preferably at a concentration of 0.5% w/w over the total of the formulation.

### 6.- Stabilizing agents

It is necessary to highlight the deletion, due to being unnecessary, in the formulation described herein, of stabilizing agents such as antioxidants based on sulphites or sodium bisulphites as it is known that they can cause sensitizing reactions in susceptible persons and another great collection of coadjuvants such as surfactants or wetting agents and taste masking agents.

### Example 2. Coating of the tablets of the ibuprofen and codeine formulations

As can be observed from the above, although various forms of the formulation can be adopted, the solid dose forms are preferred and the most preferred form is that of the single-phase tablet. Said tablets or similar are preferably formed from a tablet core according to the invention and a coating around it, preferably applied in the form of suspension, with the purpose of facilitating the swallowing and thus being able to avoid, if desired, the typical gastric irritation the two active principles object herein have. The film must have a thickness such that the quantity added of the suspension on the tablets, once the solvent is volatilized, involves a weight increase of preferably between 1-5% w/w over the unitary weight, typically 4%. Said coating preferably comprises a film based on one or several forming polymers of said film which supposes in weight over the dry total of the coating of preferably 40-80%, more preferably 50%; a plasticiser which is found in a ratio with the film-forming agent of 1:4 to 1:8, more preferably of 1:6; and, an opacifying or colouring agent, as well as any of the mixtures thereof in a ratio with the film-forming agent of preferably 1:1 to 1:4, more preferably 1:3. It is preferred that the colour is applied to the tablets during the coating process, instead of incorporating colouring agents directly on the core granules before compression.

The film-forming polymer or polymers are preferably selected from various modified cellulose ethers, such as hypromellose or hydroxypropyl methylcellulose (Shinetsu, Japan) and which avoid the direct contact of the codeine salt with the taste buds which would cause a rejection in the patient when swallowing. It is usually reinforced with the addition of an agent which avoids the polymer from cracking due to the stresses exerted on the core surface such as Macrogol 4000 or Macrogol 6000 and an opacifier such as titanium dioxide or Opaspray® white M-1-7111B from Colorcon Co. (USA). Another polymer that can be used is Sepifilm® from the company Safic-Alcan (France) based on hydroxypropyl methylcellulose, cellulose, stearic acid and titanium dioxide in the form of suspensions already prepared in water between 10-15%, in particular the types LP 770, LP 761 or, if colour is desired, in a mixture of Sepifilm® LP 761 with Sepisperse® dry 1036 blue in proportions 0.7-1:1-1.3 and other similar types, such as Sepisperse® dry 3024 yellow. A coating which can also be used is based on polymers derived from acrylic and methacrylic acids which are gastro-resistant as, given the excellent absorption throughout the gastrointestinal tract of the drug invented, it is possible to use one of them to avoid the direct contact of the preparation with the stomach removing only here its immediate dissolution and thus not producing various episodes of gastritis and ulcers frequent for this type of highly medicated patient. Said polymers are then fully dissolved in the intestine without detriment to their absorption.

The preferred coating is that marketed by the company Rohm & Haas (Germany) with the name of Eudragit® L reinforced with a plasticizing agent such as diethyl citrate and applied in the form of a 30% suspension of all the previous solids in purified water.

Surprisingly, it has been verified by additional stability experiments in accelerated storage conditions (temperature of 40°C and relative humidity of 75%) that, based on an identical formulation of uncoated cores and after being coated with the coatings described herein (yellow coating or more successfully another blue one (see example 4)), their appearance can be duly improved, masking the appearance of an unpleasant browning for the patient (Figure 12).

If desired, the tablets may later be printed using a normal food grade dye. They may also alternatively be engraved.

### Example 3. Preparation methods of the ibuprofen and codeine formulations.

The formulations may be prepared by any method known in the state of the art. For example, the single-phase tablets may be prepared by the direct compression method, wherein all the ingredients of the tablet core are screened together, mixed and later compressed. The choice of the starting formula requires a preliminary study of the diluents typically used, already mentioned above. This direct compression method avoids the need for pre-treatment of the ingredients in powder. In contrast, the wet granulation technique does require previous treatment of the ingredients in powder and a drying thereof or the dry granulation technique.

In the event that the powdered ingredients of the formulation have poor aptitude for highspeed compression processes, an additional treatment for regrouping of said ingredients is usually performed, such as said granulation technique. The wet granulation thus arises wherein the active principles, herein ibuprofen and codeine, together with the majority ingredients, mainly the diluents, are wetted with a binding solution. It has been observed that water is used as a vehicle for said dissolution, in principle, this tends to destabilize the formula, a fact which would not be remedied either by replacing it by a hydrophilic solvent, such as alcohol and its derivatives, as it would continue to need the "binding" agent that has a proven capacity of destabilizing the codeine under any of its salts.

The other alternative is "dry" granulation. In this case, the previous regrouping of the ingredients (in powder) is achieved by compacting, without the need for solvents or ligands, and it could be said that in fact a double compression is performed. Herein, said technique combined its suitable stabilization of the drugs with a compression outside of any doubts as shown in Figure 13, as the tablets are prepared by preliminary compaction of ibuprofen with pregelatinized starch in the form of typical dry granulation. The later addition of the other ingredients in the formula bearing in mind the previous observations gives rise to tablets with better compressibility than through another form of granulation or simple direct compression.

Preferably, the quantity of humidity present in the ingredients and during the processing is kept to a minimum, more preferably, the humidity content is less than approximately 3.5% w/w over the total of the formulation, since, as has already been mentioned, higher humidity percentages favour that the compression process does not occur satisfactorily.

Once the tablets are prepared in accordance with the invention, they are packaged in a suitable container such as a glass or plastic resin bottle of the type of high density polyethylene or, preferably, the so-called blister pack based on a double sheet of aluminium with a width of 20-40 µm each one sealed with heat or a sheet of vinyl chloride of 200-300 µm thickness sealed with an aluminium sheet of 20-40 µm thickness. In particular, to provide the "patient packaging" it is again packaged in a cardboard box containing the patient information leaflet with information referring to the use and dosage of the formulation.

As will be understood by persons skilled in the art, herein provided is the use of a formulation described in the invention in therapy, in particular in the treatment of chronic pain which can be present in pathologies such as: osteoarthritis, rheumatoid arthritis, ankylosing spondylitis, seronegative autoimmune diseases, bursitis, cold shoulder, De Quervain's disease or pain associated with cancer.

Therefore provided herein is the use of a formulation in accordance with this in the preparation of a drug to be used in a procedure to treat said state, the procedure comprising administering to a patient that needs it an effective quantity of a pharmaceutical formulation in accordance with the invention.

### Example 4. Formulation I of the core

The formula followed for the preparation of this formulation is that described in Table I, where the ibuprofen has an average particle size of 150 µm.

**Table I**

| **Tablet core** | **Unitary formula (mg)** | **%** |
|---|---|---|
| ibuprofen | 400 | 59.7 |
| Codeine phosphate hemihydrate | 30 | 4.5 |
| Mannitol | 118.7 | 17.7 |
| Pregelatinized starch | 72 | 10.7 |
| Colloidal anhydrous silica | 2.4 | 0.4 |
| Sodium starch glycolate | 33.5 | 5.0 |
| Stearyl fumarate Na | 13.4 | 2.0 |
| TOTAL | 670 | 100 |

The procedure carried out for the preparation of the tablet cores with the ingredients of Table I and in accordance with the present invention, was performed using the direct compression procedure, as described in Diagram I.

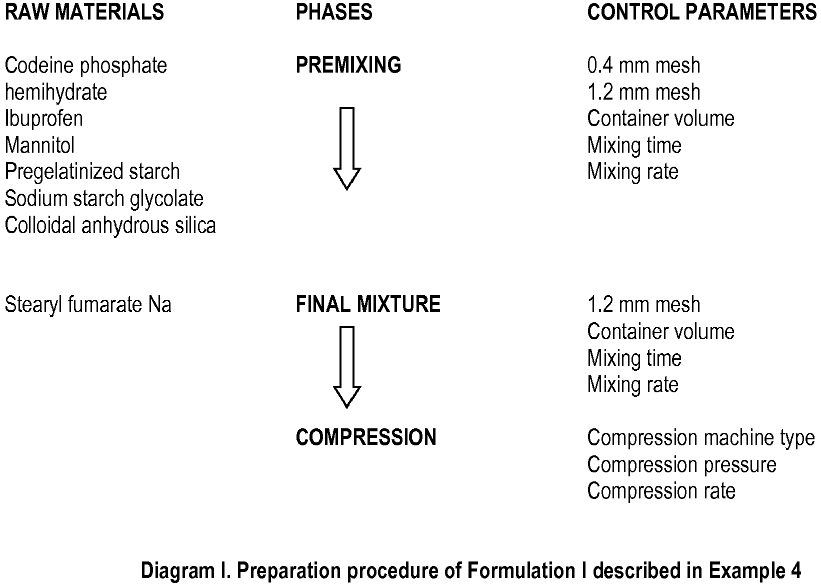

Firstly, in the premixing phase, a prior screening is performed of the raw materials through a sieve with a mesh size of 1.2 mm with the exception of the codeine phosphate hemihydrate which requires pulverization in a Stokes Tornado mill (England) and previous screening with a smaller size. Then all the raw materials are mixed in a V-type mixer except the lubricant, sodium stearyl fumarate, which is incorporated at the end during a shorter period of time to guarantee its extragranular functions. Once the mixture has been obtained, it is compressed in a Kilian RT rotary machine (Germany) with biconvex oblong punches of 16.3 x 7.2 mm.

### Example 5. Formulation II of the core

This example describes a manufacturing procedure of briquettes or compacted granules in an eccentric compression machine from Bonals BMT (Spain), using for this a round, flat mould of 20 mm in diameter. The formula followed is that described in Table II, where the ibuprofen comes from Albermarle Corporation (USA) with an average particle size of 115 (90-140 µm).

**Table II**

| **Tablet core** | **Unitary formula (mg)** | **%** | **Batch formula (g)** |
|---|---|---|---|
| Ibuprofen | 400 | 59.7 | 47760.0 |
| Codeine phosphate hemihydrate | 30 | 4.5 | 3600.0 |
| Mannitol | 122.7 | 18.3 | 14640.0 |
| Pregelatinized starch | 67 | 10.0 | 8000.0 |
| Colloidal anhydrous silica | 3.4 | 0.5 | 400.0 |
| Sodium starch glycolate | 33.5 | 5.0 | 4000.0 |
| Sodium stearyl fumarate | 13.4 | 2.0 | 1600.0 |
| TOTAL | 670 | 100 | 80000.0 |

To prepare the tablets exemplified here, the dry granulation method is used by compaction of powdered ibuprofen and pregelatinized starch and later regularization of the briquettes obtained in a Frewitt oscillating granulator (Switzerland) with a mesh size of 2 mm. Once the granulated ibuprofen has been obtained, the process shown in Diagram II is followed, the other ingredients are added and all the raw materials are mixed in a V-type mixer except the lubricant, sodium stearyl fumarate, which is incorporated at the end during a shorter period of time to guarantee its extragranular functions. Once the mixture has been obtained, it is compressed in a Kilian 26-station E-150 rotary machine (Germany) with biconvex round punches of 12 mm in diameter.

The control phases and diameters are shown in Diagram II for an industrial batch size of 80 kg.

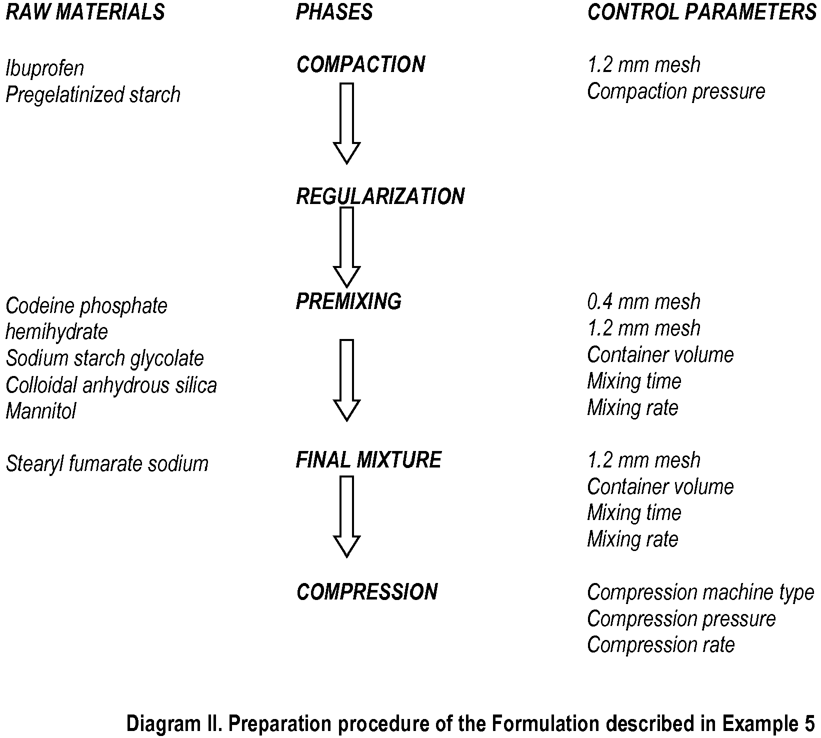

### Example 6. Formulation III core/coating.

The present example describes the preparation of cores based on ibuprofen and codeine, of the tablets according to the same ingredients and the same production process as described in Example 5, Diagram II.

Once the tablet cores were synthesized they were coated in a stainless steel coating pump, GS model 2155-HT, which is formed by an air extraction blade, a spray gun with four 1.2 mm nozzles and four no. 3 capsules (IMA, Italy). The suspension for the coating was performed based on Sepifilm® LP 761 and Sepisperse® dry 1036 blue according to the formula of Table III and the procedure described in Diagram III, giving rise to a blue-coloured coating. Said suspension is found at 13% in purified water and is applied in the form of aerosol. The tablets thus coated have a unitary weight of 696.8 mg as their original weight has increased by 4% (26.8 mg).

**Table III**

| **Tablet coating** | **% w/w suspension** | **Tablet dose (mg)** |
|---|---|---|
| Sepifilm® LP 761 | 7 | 15.4 |
| Sepisperse® dry 1036 blue | 5 | 11.4 |
| Purified water | 88 | 0.00 |
| Total | 100.00 | 26.8 |

If desired, the tablet coating colour can be changed, only modifying the ingredient Sepisperse® dry 1024 blue, for Sepisperse® dry 3024 yellow, achieving a yellow-coloured coating, or another which is white, simply removing the aforementioned Sepisperse® dry colour pigment from the formula.

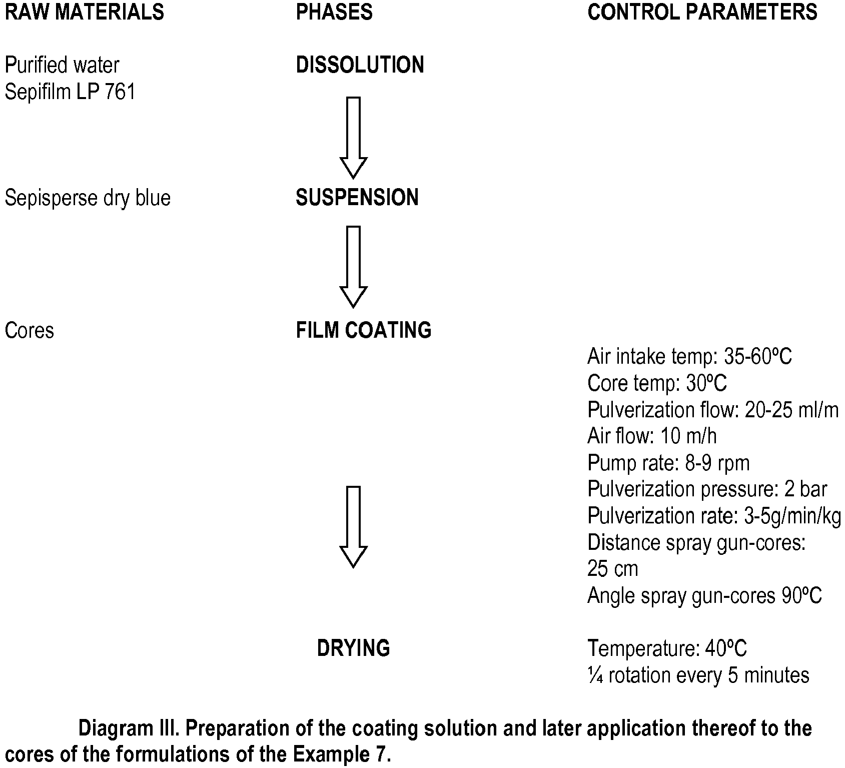

In the tablets synthesized using the procedure described in this example, the potency was analysed (richness of ibuprofen/codeine phosphate) of different formulas of ibuprofen and codeine phosphate coated and stored at 25°C and with a percentage of relative humidity of 60%. As can be observed in Figure 14 the greater potency is demonstrated of the formulations synthesized using the procedure described in Example 7 compared with the same compositions as those described in this example but wherein sodium stearyl fumarate has been eliminated from the formulation or replaced by magnesium stearate.

### Example 7. Formulation IV core/coating.

The present example describes the preparation of cores based on ibuprofen and codeine, of the tablets according to the same production process as described in Example 5 as well as their identical qualitative formula (Table III). The cores are then coated in the same apparatus of Example 6. The suspension is found at 30% in purified water applied in aerosol based on Eudragit® L 30 D-55 and other ingredients according to the formula of Table IV and the process of Diagram IV. The tablets thus coated, white in colour, have a unitary weight of > 670 mg as their original weight has increased by 2% (11.8 mg) with a gastro-resistant coating that avoids the damaging gastric effects of ibuprofen.

**Table IV**

| Tablet coating | % w/w suspension | Batch formula (kg) | Tablet dose (mg) |
|---|---|---|---|
| Eudragit® L 30 D-55 | 16.7% | 6.66 | 6.55 |
| Talc | 6.3% | 2.51 | 2.47 |
| Titanium dioxide | 4.0% | 1.60 | 1.56 |
| Triethyl citrate | 2.82 | 1.12 | 1.11 |
| Silicon antifoaming agent | 0.28% | 0.11 | 0.11 |
| Purified water | 70.0 | 28.00 | 0.00 |
| Total | 100.00 | 40.00 | 11.8 |

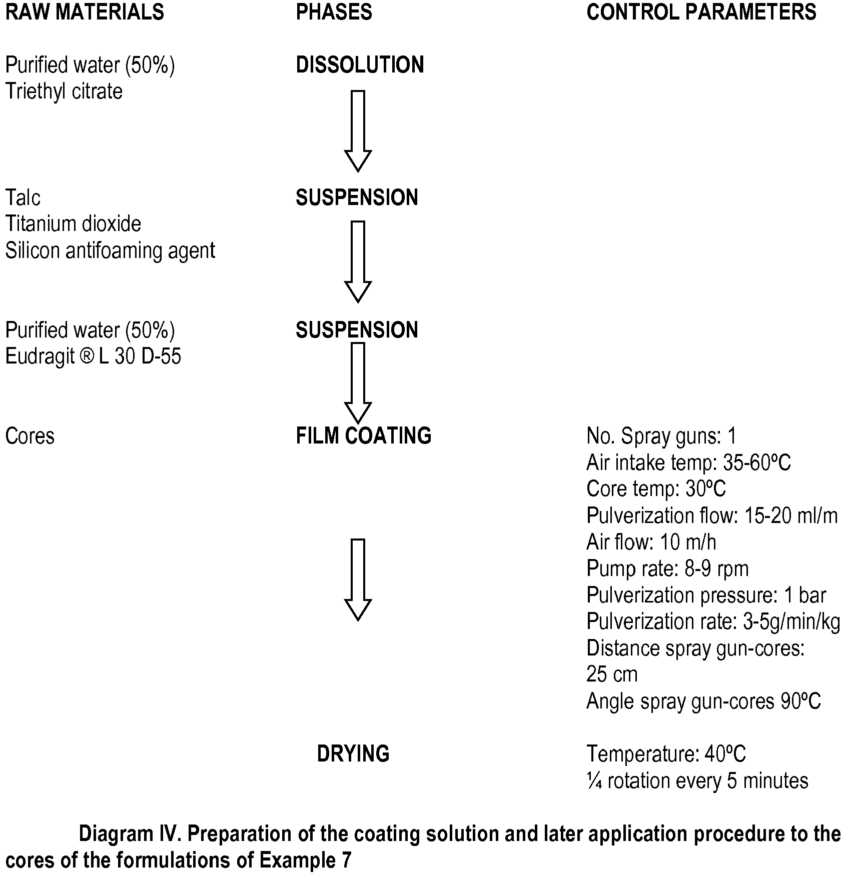

### BIBLIOGRAPHY

1. EP0388125 *Medicament.*
2. EP0535841 *Use* of a *combination of ibuprofen and codeine for the treatment of pain.*
3. Levina M et al. Drug Dev. Ind. Pharm., 2002, 28:5, 495-514
4. Roberts et al. J. Pharm. Pharmacol., 2004, 56:3, 299-305
5. US4839176.*Therapeutic agents*
6. EP0159852 *Directly compressible pharmaceutical compositions*
7. EP0220805 *Multiphase tablet and process for the preparation thereof.*
8. US4844907 *Pharmaceutical composition comprising analgesic and anti-inflammatory agent.*
9. EP0274845 *Stable solid pharmaceutical composition containing ibuprofen and codeine.*
10. EP0777477 *Improved pharmaceutical formulations containing ibuprofen and codeine*
11. Shah et al. Drug Development and Industrial Pharmacy, 1986, 12, 1329-1346)

## Claims

1. Pharmaceutical formulation in the form of non-coated tablets, coated tablets, tablets with multiple layers, lozenges, capsules or pills comprising ibuprofen or one of its pharmaceutically acceptable salts and codeine or one of its pharmaceutically acceptable salts, in combination with a pharmaceutically acceptable excipient, said excipient comprising at least: one diluent, a disintegrant, a fluidifying agent and a lubricant, **characterized in that** the diluent is mannitol, the disintegrant is sodium starch glycolate, the fluidifying agent is colloidal anhydrous silica, and the lubricant is sodium stearyl fumarate, and that the formulation additionally comprises pregelatinized starch as an additional diluent and/or disintegrant.

2. Pharmaceutical formulation according to claim 1, **characterized in that** the diluent is added at a concentration of between 10-50% w/w over the total weight of the formulation.

3. Pharmaceutical formulation according to claim 2, **characterized in that** the diluents used are mannitol at a concentration of 20% w/w over the total weight of the formulation and/or pregelatinized starch at a concentration of 10% over the total weight of the formulation.

4. Pharmaceutical formulation according to claim 1, **characterized in that** the disintegrant is added at a concentration of between 2-20% w/w over the total weight of the formulation.

5. Pharmaceutical formulation according to claim 1, **characterized in that** the fluidifying agent is added at a concentration of between 0.1-1% w/w over the total weight of the formulation.

6. Pharmaceutical formulation according to claim 1, **characterized in that** the sodium stearyl fumarate is added at a concentration ≥ 2% over the weight of the formulation.

7. Pharmaceutical formulation according to any of the preceding claims, consisting of a core coated with a film coating wherein said core comprises ibuprofen or one of its pharmaceutically acceptable salts and codeine or one of its pharmaceutically acceptable salts, in combination with a pharmaceutically acceptable excipient, said excipient comprising at least: one diluent, a disintegrant, a fluidifying agent and a lubricant, **characterized in that** the diluent is mannitol, the disintegrant is sodium starch glycolate, the fluidifying agent is colloidal anhydrous silica, and the lubricant is sodium stearyl fumarate, and that the formulation additionally comprises pregelatinized starch as an additional diluent and/or disintegrant.

8. Pharmaceutical formulation according to claim 7, **characterized in that** the film coating is formed by at least one film-forming polymer, a plasticizing agent and an opacifying or colouring agent, or mixtures thereof.

9. Pharmaceutical formulation according to claim 8, **characterized in that** the film-forming polymers are selected from modified cellulose ethers.

10. Pharmaceutical formulation according to claim 8, **characterized in that** the plasticizing agent is selected from macrogol 4000, macrogol 6000 or diethyl citrate.

11. Pharmaceutical formulation according to claim 8, **characterized in that** the opacifying or colouring agent is titanium dioxide.

12. Pharmaceutical formulation according to any of the preceding claims, comprising a concentration of 250 mg to 750 mg of ibuprofen, and a concentration of 20 mg to 40 mg of codeine as hemihydrate phosphate, per dosage unit.

13. Pharmaceutical formulation of claims 1 to 12, for use in the treatment or prophylaxis of acute and chronic pain or inflammation.

14. Preparation procedure of the formulation of claims 1 to 12 **characterized in** a direct compression method which consists of a premixing phase, comprising:
a) Pulverization and screening of the codeine.
b) Screening of the ibuprofen together with the excipients, with the exception of the lubricant, through a sieve with appropriate mesh size
and a mixing phase, comprising:
c) Mixing the pulverized and screened codeine in the previous step together with the other ingredients, ibuprofen and excipients, with the exception of the lubricant, in an appropriate mixer.
d) Adding the lubricant and compress the mixture obtained.
e) Optionally, coating the compressed tablet cores obtained in the previous stage with a film.

15. Preparation procedure of the formulation of claims 1 to 12 **characterized in** a dry granulation method which consists of:
a) a compaction phase comprising mixing the ibuprofen with a disintegrant and submitting them to a suitable pressure obtaining briquettes, and furthermore,
b) a regularization phase comprising submitting the briquettes obtained in the previous step to a regularization procedure in a regularizing machine until obtaining granulated ibuprofen,
c) a premixing phase comprising adding the codeine and the other excipients, with the exception of the lubricant, to the granulated ibuprofen obtained in the regularization phase and then screening it,
d) a mixing phase comprising the addition of the lubricant to the mixture obtained in the premixing phase and
e) a compression phase comprising the compression of the mixture obtained in the mixing phase in a compression machine
f) optionally, coating the compressed tablet cores obtained in the previous stage by the application of a suspension.

## Patentansprüche

1. Pharmazeutische Formulierung in Form von nicht überzogenen Tabletten, überzogenen Tabletten, Tabletten mit mehreren Schichten, Lutschtabletten, Kapseln oder Pillen, umfassend Ibuprofen oder eines seiner pharmazeutisch verträglichen Salze und Codein oder eines seiner pharmazeutisch verträglichen Salze in Kombination mit einem pharmazeutisch verträglichen Hilfsstoff, der Hilfsstoff mindestens umfassend: ein Verdünnungsmittel, ein Sprengmittel, ein Verflüssigungsmittel und ein Schmiermittel, **dadurch gekennzeichnet, dass** das Verdünnungsmittel Mannitol, das Sprengmittel Natriumstärkeglykolat, das Verflüssigungsmittel kolloidales wasserfreies Siliciumdioxid und das Schmiermittel Natriumstearylfumarat ist und dass die Formulierung zusätzlich vorgelatinierte Stärke als zusätzliches Verdünnungsmittel und/oder Sprengmittel umfasst.

2. Pharmazeutische Formulierung nach Anspruch 1, **dadurch gekennzeichnet, dass** das Verdünnungsmittel in einer Konzentration zwischen 10-50 % Gew./Gew. über das Gesamtgewicht der Formulierung zugegeben wird.

3. Pharmazeutische Formulierung nach Anspruch 2, **dadurch gekennzeichnet, dass** die verwendeten Verdünnungsmittel Mannitol in einer Konzentration von 20 % Gew./Gew. über das Gesamtgewicht der Formulierung und/oder vorgelatinierte Stärke in einer Konzentration von 10 % über das Gesamtgewicht der Formulierung sind.

4. Pharmazeutische Formulierung nach Anspruch 1, **dadurch gekennzeichnet, dass** das Sprengmittel in einer Konzentration zwischen 2-20 % Gew./Gew. über das Gesamtgewicht der Formulierung zugegeben wird.

5. Pharmazeutische Formulierung nach Anspruch 1, **dadurch gekennzeichnet, dass** das Verflüssigungsmittel in einer Konzentration zwischen 0,1-1 % Gew./Gew. über das Gesamtgewicht der Formulierung zugegeben wird.

6. Pharmazeutische Formulierung nach Anspruch 1, **dadurch gekennzeichnet, dass** das Natriumstearylfumarat in einer Konzentration von ≥ 2 % über das Gesamtgewicht der Formulierung zugegeben wird.

7. Pharmazeutische Formulierung nach einem der vorhergehenden Ansprüche, bestehend aus einem Kern, der mit einem Filmüberzug überzogen ist, wobei der Kern Ibuprofen oder eines seiner pharmazeutisch verträglichen Salze und Codein oder eines seiner pharmazeutisch verträglichen Salze in Kombination mit einem pharmazeutisch verträglichen Hilfsstoff umfasst, der Hilfsstoff mindestens umfassend: ein Verdünnungsmittel, ein Sprengmittel, ein Verflüssigungsmittel und ein Schmiermittel, **dadurch gekennzeichnet, dass** das Verdünnungsmittel Mannitol, das Sprengmittel Natriumstärkeglykolat, das Verflüssigungsmittel kolloidales wasserfreies Siliciumdioxid und das Schmiermittel Natriumstearylfumarat ist, und dass die Formulierung zusätzlich vorgelatinierte Stärke als zusätzliches Verdünnungsmittel und/oder Sprengmittel umfasst.

8. Pharmazeutische Formulierung nach Anspruch 7, **dadurch gekennzeichnet, dass** die Filmüberzug aus mindestens einem filmbildenden Polymer, einem Weichmacher und einem Trübungs- oder Färbemittel oder Mischungen davon gebildet wird.

9. Pharmazeutische Formulierung nach Anspruch 8, **dadurch gekennzeichnet, dass** die filmbildenden Polymere aus modifizierten Celluloseethern ausgewählt sind.

10. Pharmazeutische Formulierung nach Anspruch 8, **dadurch gekennzeichnet, dass** der Weichmacher aus Macrogol 4000, Macrogol 6000 oder Diethylcitrat ausgewählt ist.

11. Pharmazeutische Formulierung nach Anspruch 8, **dadurch gekennzeichnet, dass** das Trübungs- oder Färbemittel Titandioxid ist.

12. Pharmazeutische Formulierung nach einem der vorhergehenden Ansprüche, umfassend eine Konzentration von 250 mg bis 750 mg Ibuprofen und eine Konzentration von 20 mg bis 40 mg Codein als Halbhydratphosphat, pro Dosierungseinheit.

13. Pharmazeutische Formulierung nach den Ansprüchen 1 bis 12, zur Verwendung bei der Behandlung oder Prophylaxe von akuten und chronischen Schmerzen oder Entzündungen.

14. Herstellungsverfahren der Formulierung nach den Ansprüchen 1 bis 12, **gekennzeichnet durch** eine direkte Kompressionsmethode, die aus einer Vormischphase besteht, umfassend:
a) Pulverisieren und Sieben des Codeins.
b) Sieben des Ibuprofens zusammen mit den Hilfsstoffen, mit Ausnahme des Schmiermittels, durch ein Sieb mit geeigneter Maschenweite und einer Mischphase, umfassend:
c) Mischen des im vorhergehenden Schritt pulverisierten und gesiebten Codeins zusammen mit den anderen Zutaten, Ibuprofen und Hilfsstoffen, mit Ausnahme des Schmiermittels, in einem geeigneten Mischer.
d) Zugabe des Schmiermittels und Komprimieren der erhaltenen Mischung.
e) Optional, Überziehen der in der vorhergehenden Stufe erhaltenen komprimierten Tablettenkerne mit einem Film.

15. Herstellungsverfahren für die Formulierung nach den Ansprüchen 1 bis 12, **gekennzeichnet durch** eine Trockengranulationsmethode, bestehend aus:
a) einer Verdichtungsphase, umfassend das Mischen des Ibuprofens mit einem Sprengmittel und das Beaufschlagen derselben mit einem geeigneten Druck, wobei Briketts erhalten werden, und darüber hinaus,
b) einer Regularisierungsphase, umfassend das Unterziehen der im vorhergehenden Schritt erhaltenen Briketts einem Regularisierungsverfahren in einer Regularisierungsmaschine, bis granuliertes Ibuprofen erhalten wird,
c) einer Vormischungsphase, umfassend die Zugabe des Codeins und der anderen Hilfsstoffe, mit Ausnahme des Schmiermittels, zu dem in der Regularisierungsphase erhaltenen granulierten Ibuprofen und anschließendes Sieben,
d) einer Mischphase, umfassend die Zugabe des Schmiermittels zu dem in der Vormischphase erhaltenen Gemisch und
e) einer Kompressionsphase, umfassend die Kompression des in der Mischphase erhaltenen Gemisches in einer Kompressionsmaschine
f) optional, Überziehen der in der vorhergehenden Stufe erhaltenen komprimierten Tablettenkerne durch Aufbringen einer Suspension.

## Revendications

1. Préparation pharmaceutique sous forme de comprimés non enrobés, de comprimés enrobés, de comprimés multicouches, de pastilles, de capsules ou de gélules comprenant de l'ibuprofène ou l'un de ses sels pharmaceutiquement acceptables et de la codéine ou l'un de ses sels pharmaceutiquement acceptables, en combinaison avec un excipient pharmaceutiquement acceptable, ledit excipient comprenant au moins : un diluant, un désintégrant, un agent fluidifiant et un lubrifiant, **caractérisée en ce que** le diluant est le mannitol, le désintégrant est le glycolate d'amidon sodique, l'agent fluidifiant est la silice colloïdale anhydre, et le lubrifiant est le fumarate de stéaryle sodique, et **en ce que** la préparation comprend également de l'amidon prégélatinisé en tant que diluant et/ou désintégrant supplémentaire.

2. Préparation pharmaceutique selon la revendication 1, **caractérisée en ce que** le diluant est ajouté à une concentration comprise entre 10 % et 50 % en poids du poids total de la préparation.

3. Préparation pharmaceutique selon la revendication 2, **caractérisée en ce que** les diluants utilisés sont le mannitol à une concentration de 20 % en poids par rapport au poids total de la préparation et/ou l'amidon prégélatinisé à une concentration de 10 % en poids du poids total de la préparation.

4. Préparation pharmaceutique selon la revendication 1, **caractérisée en ce que** le désintégrant est ajouté à une concentration comprise entre 2 % et 20 % en poids du poids total de la préparation.

5. Préparation pharmaceutique selon la revendication 1, **caractérisée en ce que** l'agent fluidifiant est ajouté à une concentration comprise entre 0,1 % et 1 % en poids du poids total de la préparation.

6. Préparation pharmaceutique selon la revendication 1, **caractérisée en ce que** le fumarate de stéaryle sodique est ajouté à une concentration ≥ 2 % du poids de la préparation.

7. Préparation pharmaceutique selon l'une des revendications précédentes, consistant en un noyau enrobé d'un film d'enrobage, dans laquelle ledit noyau comprenant de l'ibuprofène ou l'un de ses sels pharmaceutiquement acceptables et de la codéine ou l'un de ses sels pharmaceutiquement acceptables, en combinaison avec un excipient pharmaceutiquement acceptable, ledit excipient comprenant au moins : un diluant, un désintégrant, un agent fluidifiant et un lubrifiant, **caractérisée en ce que** le diluant est le mannitol, le désintégrant est le glycolate d'amidon sodique, l'agent fluidifiant est la silice colloïdale anhydre, et le lubrifiant est le fumarate de stéaryle sodique, et **en ce que** la préparation comprend également de l'amidon prégélatinisé en tant que diluant et/ou désintégrant supplémentaire.

8. Préparation pharmaceutique selon la revendication 7, **caractérisée en ce que** le film d'enrobage est formé d'au moins un polymère filmogène, un agent plastifiant et un agent opacifiant ou colorant, ou des mélanges de ces derniers.

9. Préparation pharmaceutique selon la revendication 8, **caractérisée en ce que** les polymères filmogènes sont sélectionnés parmi des éthers de cellulose modifiés.

10. Préparation pharmaceutique selon la revendication 8, **caractérisée en ce que** l'agent plastifiant est sélectionné parmi le macrogol 4000, le macrogol 6000 ou le citrate diéthylique.

11. Préparation pharmaceutique selon la revendication 8, **caractérisée en ce que** l'agent opacifiant ou colorant est le dioxyde de titane.

12. Préparation pharmaceutique selon l'une des revendications précédentes, comprenant une concentration d'ibuprofène comprise entre 250 mg et 750 mg, et une concentration de codéine en tant que phosphate hémihydraté comprise entre 20 mg et 40 mg, par unité de prise.

13. Préparation pharmaceutique selon les revendications 1 à 12, destinée à être utilisée dans le traitement ou la prophylaxie des douleurs ou inflammations aiguës ou chroniques.

14. Procédure d'élaboration de la préparation selon les revendications 1 à 12, **caractérisée en ce qu'**un procédé de tassage direct comprenant une phase de mélange préalable consistant à :
a) Pulvériser et filtrer la codéine.
b) Filtrer l'ibuprofène ainsi que les excipients, à l'exception du lubrifiant, via un tamis au maillage adéquat et une phase de mélange consistant à :
c) Mélanger la codéine pulvérisée et filtrée dans l'étape précédente avec les autres ingrédients, l'ibuprofène et les excipients, à l'exception du lubrifiant, dans un mixeur adéquat.
d) Ajouter le lubrifiant et tasser le mélange obtenu.
e) Facultativement, enrober les noyaux de comprimés tassés obtenus à l'étape précédente avec un film.

15. Procédure d'élaboration de la préparation selon les revendications 1 à 12, **caractérisée en ce qu'**un procédé de granulation à sec comprenant :
a) une phase de compactage consistant à mélanger l'ibuprofène avec un désintégrant et à les soumettre à une pression adaptée afin d'obtenir des briquettes, puis
b) une phase de régularisation consistant à soumettre les briquettes obtenues à l'étape précédente à une procédure de régularisation dans une machine de régularisation jusqu'à l'obtention de l'ibuprofène sous forme de granulés,
c) une phase de mélange préalable consistant à ajouter la codéine et les autres excipients, à l'exception du lubrifiant, à l'ibuprofène sous forme de granulés obtenu lors de la phase de régularisation, et à le filtrer,
d) une phase de mélange consistant à ajouter le lubrifiant au mélange obtenu lors de la phase de mélange préalable et
e) une phase de tassage consistant à tasser le mélange obtenu lors de la phase de mélange dans une machine de tassage
e) facultativement, une phase d'enrobage des noyaux de comprimés tassés obtenus à l'étape précédente en appliquant une suspension.
